# EUROPÄISCHE PATENTSCHRIFT

(11) **EP 1 432 465 B1**
(45) Veröffentlichungstag und Bekanntmachung des Hinweises auf die Patenterteilung: **07.12.2005**
(21) Anmeldenummer: 02754095.4
(22) Anmeldetag: 26.08.2002
(51) Int. Cl.: A61M 5/24

(54) **IDENTIFICATION FROM AMPOULE**
VIAL IDENTIFICATION
IDENTIFICATION D'AMPOULE

(30) Priorität: 28.09.2001 DE 10147973
(43) Veröffentlichungstag der Anmeldung: 30.06.2004
(73) Patentinhaber: TecPharma Licensing AG, 3401 Burgdorf (CH)
(72) Erfinder: STEFFEN, Beat, CH-3792 Saanen (CH); YEANDEL, Julian, CH-3506 Grosshöchstetten (CH); PONZER, Rainer, CH-3414 Oberburg (CH); JOST, Stefan, CH-3201 Mühleberg (CH); SEBASTE, Alessandro, CH-4054 Basel (CH); EICH, Adrian, CH-3374 Wangenried (CH)
(86) Internationale Anmeldenummer: PCT/CH2002/000465
(87) Internationale Veröffentlichungsnummer: WO 2003/028790

(56) Entgegenhaltungen:
- EP-A- 1 095 668
- WO-A-01/56635
- WO-A-98/00187
- US-A- 5 681 285
- US-A- 6 042 571
- US-A- 6 110 152

## Beschreibung

Die vorliegende Erfindung bezieht sich auf eine Ampulle, bevorzugt mit einem darin enthaltenen Produktfluid, für ein Injektions- oder Infusionsgerät sowie ein solches Injektions- oder Infusionsgerät mit einer Ampullenerkennung.

Vorrichtungen zur Verabreichung von Produktfluiden aus Ampullen sind in der Form von tragbaren Infusions- und Injektionsgeräten bekannt, welche beispielsweise bei der Insulinbehandlung eingesetzt werden. Dabei werden Ampullen, welche mit der zu verabreichenden Substanz gefüllt sind, mit einer Verabreichungsvorrichtung - oft auch als Pen bezeichnet - gekoppelt, um die in der Ampulle enthaltene Substanz über die Verabreichungsvorrichtung an einen Patienten abzugeben. Es gibt eine Vielzahl von Substanzen welche auf diese Art verabreicht werden, wie zum Beispiel Präparate mit Insulin bei Zuckerkrankheit, Wachstumshormonen (hGH; human Growth Hormon) bei Wachstumsstörungen, Erythropoietin (Epo) bei Niereninsuffizienz oder allgemeinem Mangel an roten Blutkörperchen, α-Interferon zum Beispiel bei der Hepatitis- oder Krebsbehandlung oder potenzfördernde Mittel. Dabei werden die geometrisch oft identischen Ampullen häufig mit verschiedenen Substanzen oder auch mit unterschiedlichen Konzentrationen einer zu verabreichenden Substanz gefüllt.

Um die Gefahr einer Verwechslung von Behältern mit unterschiedlichen Substanzen zu verringern, sind unterschiedlich ausgebildete Verabreichungsvorrichtungen bekannt, in welche die jeweiligen zugehörigen Behälter eingesteckt werden können.

Aus der WO 98/00187 ist ein Behälter mit einer darauf anbringbaren Farbcodierung, bestehend aus mehreren unterschiedlichen Farbfeldern bekannt, wobei eine Eigenschaft eines Behälters oder sein Inhalt mittels eines optischen Sensorsystems erkannt werden kann.

Aus der WO 01/56635 A1 der Anmelderin sind ein Behälter und eine Vorrichtung zur Verabreichung einer Substanz bekannt, wobei dem Behälter ein Erkennungselement zugeordnet ist.

Es ist eine Aufgabe der vorliegenden Erfindung eine Ampulle und eine Verabreichungsvorrichtung vorzuschlagen, wobei verschiedene Ampullen-Typen automatisch von der Verabreichungsvorrichtung erkannt und unterschieden werden können und wobei eine Fehl- oder Falscherkennung weitgehend ausgeschlossen ist.

Diese Aufgabe wird durch die Gegenstände der unabhängigen Ansprüche gelöst. Vorteilhafte Ausführungsformen ergeben sich aus den abhängigen Ansprüchen.

Der Begriff "Ampulle" bezeichnet einen Behälter, der zur Aufnahme einer Substanz geeignet ist. Der Behälter bzw. die Ampulle kann aus einem starren oder steifen Material sein, wie z. B. Kunststoff, Glas, Metall oder kann aus einem Material sein, welches flexibel oder elastisch ist, wie z. B. eine Folie aus Kunststoff oder einem anderen Material. Die Kanüle kann z. B. rotationssymmetrisch, zylinderförmig, quaderförmig oder beutelförmig sein und allgemein eine beliebige Geometrie aufweisen, solange innerhalb der Kanüle ein bestimmtes auch variables Volumen von z. B. 0,1-1000 ml vorhanden ist.

Die erfindungsgemäße Ampulle, welche bevorzugt etwa zylinderförmig und für eine Verwendung in einem Injektions- oder Infusionsgerät geeignet ist und bevorzugt eine zu verabreichende Substanz, insbesondere ein Produktfluid zur Selbstverabreichung aufnehmen kann, weist erfindungsgemäß mindestens zwei Erkennungselemente auf, welche an der Ampulle angeordnet oder fest mit der Ampulle verbunden sind. Die mindestens zwei Erkennungselemente können jeweils in einer von mindestens zwei, bevorzugt drei, vier oder mehr, zum Beispiel fünf bis einhundert vorgegebenen Positionen an der Ampulle angeordnet bzw. angebracht werden, wobei die mindestens zwei erfindungsgemäß vorgegebenen Positionen asymmetrisch auf oder an der Ampulle angeordnet sind. Mit anderen Worten kann ein erstes Erkennungselement in einer ersten von zum Beispiel vier vorgegebenen Positionen angebracht werden und ein zweites Erkennungselement kann an einer der dann verbleibenden drei Positionen angeordnet werden. So kann durch das erfindungsgemäße Vorsehen einer Vielzahl von vorgegebenen Positionen für mindestens zwei Erkennungselemente eine eindeutige Identifikation eines bestimmten Ampullen-Typs vorgenommen werden, indem zum Beispiel für eine Ampulle, welche mit einer ersten Substanz bei einer ersten Konzentration gefüllt ist, die Erkennungselemente an zwei bestimmten der vorgegebenen Positionen angebracht werden und bei einer anderen Ampulle mit einer anderen zu verabreichenden Substanz oder zum Beispiel der gleichen Substanz mit einer anderen Konzentration zwei Erkennungselemente an davon verschiedenen der vorgegebenen Positionen an der Ampulle angebracht werden. Die vorgegebenen Positionen sind erfindungsgemäß asymmetrisch an der Ampulle angeordnet, d. h. zum Beispiel nicht rotationssymmetrisch oder z. B. bei Vorgeben von vier verschiedenen auf einem Kreis liegenden Positionen zum Anbringen der Erkennungselemente ist der Winkelabstand zwischen jeweils zwei beliebigen Positionen bevorzugt nicht gleich dem Winkelabstand zwischen zwei anderen Positionen. Somit kann eine eindeutige Identifikation der Ampulle erfolgen, wobei zusätzlich die Fehlersicherheit bei der Erkennung erhöht wird, da z. B. bei einem Verdrehen der Ampulle aufgrund der Asymmetrie keine falsche Bestimmung eines durch die Anordnung der Erkennungselemente kodierten Ampulleninhalts möglich ist. Tritt zum Beispiel ein Fehler auf, indem beispielsweise eines der mindestens zwei Erkennungselemente fehlerhaft oder aus der Ampulle herausgefallen ist oder beispielsweise ein Defekt an der Ampulle vorliegt, welcher wie ein drittes Erkennungselement wirkt, so kann ein solcher Fehler durch die erfindungsgemäße Ampullenerkennung identifiziert und gegebenenfalls sogar korrigiert werden. Der Begriff "asymmetrisch" im Sinne der Erfindung soll so verstanden werden, dass durch Verschieben bzw. Verdrehen der Ampulle mit z. B. zwei Erkennungselementen in bestimmten zwei von vier möglichen vorgegebenen Positionen keine Anordnung der mindestens zwei Erkennungselemente auf der Ampulle erhalten werden kann, welche einer Anordnung der zwei Erkennungselemente auf der Ampulle bei beliebigen zwei anderen der vier möglichen vorgegebenen Positionen entspricht.

Werden die vorgegebenen möglichen Positionen für anzubringende Erkennungselemente asymmetrisch, also z. B. nicht rotationssymmetrisch angeordnet, kann verhindert werden, dass z. B. bei einem falschen Eindrehen einer Ampulle ein falsches Signal gelesen wird.

Vorteilhaft sind mehr Positionen vorgegeben, als Erkennungselemente verwendet werden, wobei vorteilhaft zur Kennzeichnung eines Satzes verschiedener Ampullen immer die gleiche Anzahl von z. B. zwei Erkennungselementen an jeweils verschiedenen Positionen bzw. einer Kombination von Positionen verwendet wird.

Bevorzugt sind die mindestens zwei vorgegebenen Positionen oder Erkennungselemente an einem Ampullenende vorgesehen, also zum Beispiel an der Ampullenvorderseite, mit welcher die Ampulle in ein Injektions- oder Infusionsgerät eingeschoben wird. Besonders bevorzugt sind die vorgegebenen Positionen oder Erkennungselemente im Wesentlichen entlang eines Kreises angeordnet, wobei der Kreis bevorzugt konzentrisch zu einer Mittelachse oder Symmetrieachse der Ampulle ist.

Vorteilhaft sind mindestens drei vorgegebene Positionen an der Ampulle vorgegeben, an welchen die mindestens zwei Erkennungselemente angebracht werden können. Vorteilhaft können auch vier, fünf bis fünfzig oder mehr Positionen zum Anbringen von mindestens zwei Erkennungselementen vorgesehen sein.

Es können auch mehr als zwei Erkennungselemente, zum Beispiel drei bis fünfzig oder mehr Erkennungselemente an der Ampulle vorgesehen sein, um eine entsprechende Vielzahl von unterschiedlichen Ampullen-Typen oder Ampullenfüllungen identifizieren zu können und um eine möglichst hohe Fehlersicherheit oder Fehlertoleranz zu erzielen.

Besonders bevorzugt ist mindestens eines der mindestens zwei Erkennungselemente immer an einer bestimmten vorgegebenen Position fest angeordnet, d. h. diese vorgegebene Position ist immer mit einem Erkennungselement besetzt, welches als Referenz-Erkennungselement dienen kann. Die relative Anordnung des mindestens einen anderen Erkennungselements zu diesem Referenz-Erkennungselement kann dann zur eindeutigen Identifikation der Ampulle bzw. deren Inhalt verwendet werden.

Es können zusätzlich zur oben beschriebenen Anordnung von mindestens zwei Erkennungselementen an jeweils einer von mindestens zwei, bevorzugt drei oder vier vorgegebenen Positionen noch mindestens zwei, bevorzugt drei oder vier weitere Referenz-Erkennungselemente vorgesehen werden, welche zum Beispiel auf einem Kreis, bevorzugt mit etwa gleichem Winkelabstand, angeordnet sind. Dieser Kreis liegt vorteilhaft konzentrisch zu einem Kreis, in welchem die oben beschriebenen vorgegebenen Positionen für die mindestens zwei Erkennungselemente liegen. Dabei können die Referenz-Erkennungselemente innerhalb oder außerhalb dieses Kreises der oben beschriebenen Erkennungselemente angeordnet sein. Eine solche Ausgestaltung der Erfindung ermöglicht es zum Beispiel durch Detektion der bevorzugt in einem gleichen Winkelabstand entlang eines Kreises angeordneten Referenz-Erkennungselemente eine Drehbewegung der Ampulle, zum Beispiel beim Einschrauben der Ampulle in ein Injektions- oder Infusionsgerät, zu erkennen und basierend auf dieser erkannten Drehbewegung zu detektieren, in welchen der vorgegebenen Positionen die mindestens zwei Erkennungselemente in bzw. an der Ampulle angeordnet sind. Somit kann zum Beispiel bei einem Einschrauben einer Ampulle von Hand in ein Injektionsgerät trotz einer ungleichmäßigen Einschraubbewegung die Anordnung der mindestens zwei Erkennungselemente detektiert werden. Sind z. B. vier Referenz-Erkennungselemente im Winkelabstand von 90° angeordnet und erzeugen so jede Vierteldrehung ein Signal, so kann z. B. erkannt werden, ob die Erkennungselemente an zwei gegenüberliegenden oder zwei mit kleinem Winkel beanstandeten vorgegebenen Positionen liegen.

Vorteilhaft basiert das Erkennungselement auf einem elektrischen und/oder magnetischen bzw. induktiven und/oder kapazitiven und/oder mechanischen Prinzip. Als Erkennungselement kann erfindungsgemäß ein Magnet, wie zum Beispiel ein hartmagnetisches Material oder ein Dauermagnet, ein weichmagnetisches oder magnetisierbares Material oder auch ein magnetischer Kunststoff bzw. ein Kunststoff, welcher magnetische Materialien enthält, verwendet werden. Das Erkennungselement kann ergänzend hierzu oder auch alternativ zum Beispiel als leitfähige Struktur, als optische Struktur, als Oberflächenstruktur, als Schwingkreis oder als Chip ausgebildet sein. Allgemein soll das Erkennungselement so ausgebildet sein, dass das Vorhandensein oder Fehlen eines Erkennungselements an einer bestimmten Stelle detektiert werden kann. Zum Beispiel können auch verschiedene Erkennungselemente verwendet werden, welche unterschiedlich starke oder unterschiedlich gepolte elektrische und/oder magnetische Felder erzeugen, um so z. B. durch die Ausrichtung bzw. Polung und/oder Stärke eines Feldes ein bestimmtes einzelnes Erkennungselement oder eine bestimmte Gruppe von Erkennungselementen eindeutig detektieren zu können und somit diese Erkennungselemente von anderen unterscheiden zu können.

Bevorzugt kann die Eigenschaft eines Erkennungselements verändert werden, also zum Beispiel ein magnetisierbares Material in der Magnetisierungsrichtung umgepolt werden, um ein geeignetes Programmieren der Erkennungselemente einer Ampulle zu ermöglichen, so dass zum Beispiel eine Information bezüglich des Ampulleninhalts in die Erkennungselemente geschrieben werden kann.

Die erfindungsgemäße Verabreichungsvorrichtung, also zum Beispiel ein Injektions- oder Infusionsgerät, in welche die oben beschriebene Ampulle mit den mindestens zwei Erkennungselementen eingebracht, zum Beispiel eingeschraubt werden kann, ist mit mindestens einer Ampulle koppelbar und weist mindestens zwei Sensoren, bevorzugt drei, vier, fünf bis fünfzig oder mehr Sensoren an vorgegebenen Positionen auf, um mit diesen Sensoren das Vorliegen oder Fehlen eines Erkennungselementes im Bereich des Sensors, also zum Beispiel vor dem Sensor, detektieren zu können.

Gemäß einem alternativen Aspekt der vorliegenden Erfindung weist eine Verabreichungsvorrichtung mindestens einen Sensor auf, welcher zum Beispiel mittels eines Motors entlang einer in die Verabreichungsvorrichtung eingebrachten Ampulle, also zum Beispiel um die Ampulle herum oder an der Ampulle entlang bewegt werden kann, um hierdurch die relative Lage der mindestens zwei Erkennungselemente der Ampulle zu detektieren.

Wird die Ampulle zum Beispiel in die Verabreichungsvorrichtung eingeschraubt, sind die Gewinde vorteilhaft ausgerichtet, d. h. die Gewinde weisen eine möglichst enge Toleranz auf, so dass die Ampulle im vollständig eingeschraubten Zustand ein vorgegebenes bestimmtes Lageverhältnis aufweist, also zum Beispiel ein bestimmter Punkt an der Vorderseite der Ampulle im vollständig eingeschraubten Zustand an einer vorgegebenen Position liegt bzw. vorteilhaft nur möglichst wenig seitlich bezüglich der gewünschten Position verdreht ist.

Vorteilhaft kann ein Endanschlag, wie zum Beispiel ein Vorsprung oder Nocken vorgesehen sein, so dass eine Ampulle beim Einschieben oder Einschrauben an diesem Endanschlag in Anlage kommt und nicht mehr weiter gedreht werden kann, um so die Ampullenlage festzulegen.

Vorteilhaft ist mindestens ein Positionierungselement in der Verabreichungsvorrichtung vorgesehen, mit welcher der mindestens eine Sensor bzw. die Sensoren einzeln oder zusammen, zum Beispiel auf einer Leiterplatte, geeignet positioniert werden können. Dabei liegen die Sensoren vorzugsweise den vorgegebenen Positionen für die mindestens zwei Erkennungselemente der Ampulle gegenüber, wenn die Ampulle in die Verabreichungsvorrichtung eingebracht ist. Dies kann zum Beispiel an der Vorderseite der Ampulle und/oder auch an seitlichen Bereichen der Ampulle sein.

Es ist erfindungsgemäß auch möglich die Sensoren in einem gewissen Abstand von den jeweiligen Erkennungselementen bei eingesetzter Ampulle vorzusehen, wobei geeignete Übertragungselemente, wie zum Beispiel weichmagnetische Materialien zum Umlenken von Magnetfeldern, Lichtleiter oder elektrische Leiter zum Leiten von für die jeweiligen Erkennungselemente spezifischen physikalischen Eigenschaften bzw. Signalen zu den jeweiligen Sensoren vorgesehen sein können.

Die Sensoren können zum Beispiel als Hall-Sensoren, Lichtdetektoren, mechanische Schalter zum Detektieren von Oberflächenstrukturen, elektrische Schaltungen zum Bestimmen eines elektrischen Widerstandes, einer Kapazität, einer Induktivität oder einer Resonanzfrequenz oder andere geeignete Detektoren sein, welche zum Detektieren der jeweils verwendeten Erkennungselemente geeignet sind.

Es kann erfindungsgemäß ein Motor zum Einbringen der Ampulle in die Verabreichungsvorrichtung vorgesehen sein, wie zum Beispiel ein Motor zum Einschieben oder Eindrehen der Ampulle. Wird zum Beispiel die Ampulle durch einen solchen Motor beim Eindrehen mit einer bekannten, vorzugsweise konstanten Geschwindigkeit gedreht, so können zum Beispiel mittels eines einzigen fest in der Verabreichungsvorrichtung angeordneten Sensors die Positionen der mindestens zwei an der Ampulle angeordneten Erkennungselemente bereits beim Einbringen in die Verabreichungsvorrichtung detektiert werden, wodurch es bereits beim Einbringen der Ampulle in die Verabreichungsvorrichtung möglich ist, den Typ bzw. Inhalt der verwendeten Ampulle zu identifizieren.

Vorteilhaft ist mindestens ein Multiplexer vorgesehen, welcher mit mindestens zwei, bevorzugt allen der verwendeten Sensoren verschaltet ist, um die Anzahl der von den Sensoren zu einer Auswerteelektronik zu übertragenden Signale reduzieren zu können. Werden zum Beispiel vier Sensoren verwendet, so können über einen Multiplexer die vier von den Sensoren aufgenommenen Signale zum Beispiel im Zeit-Multiplexverfahren auf zwei oder auch nur auf eine einzige Leitung gegeben werden, so dass insgesamt nur eine einzige Signalleitung erforderlich ist, was den Verdrahtungsaufwand verringert.

Vorteilhaft ist an der Verabreichungsvorrichtung eine Anzeige angebracht, mit welcher der mittels der Sensoren detektierte Ampullen-Typ oder Ampulleninhalt angezeigt werden kann, um es zum Beispiel einem Benutzer zu ermöglichen, das Detektionsergebnis zu überprüfen.

Gemäß einem weiteren Aspekt der Erfindung ist die oben beschriebene Ampulle mit einer oben beschriebenen Verabreichungsvorrichtung zu einem Verabreichungssystem kombiniert.

Die Erfindung wird nachfolgend anhand bevorzugter Ausführungsformen unter Bezugnahme auf verschiedene Einzelaspekte der Erfindung beschrieben werden.

Es zeigen:
- Figur 1: eine perspektivische Ansicht einer erfindungsgemäßen Ampulle, welche in einen Pen eingesetzt ist;
- Figuren 2A und 2B: Ansichten einer Leiterplatte mit darauf angebrachten Sensoren;
- Figur 3: schematisch die in Figur 2A gezeigte Leiterplatte zur Erläuterung der asymmetrischen Anordnung der Sensoren;
- Figur 4: eine vorteilhafte Ausführungsform der erfindungsgemäßen Anordnung von Erkennungselementen und Sensoren;
- Figuren 5A und 5B: eine schematische Erläuterung des Funktionsprinzips der Magnet-Detektion;
- Figur 6: eine Ausführungsform der Erfindung mit Referenz-Erkennungselementen; und
- Figuren 7 bis 9: beispielhafte Verbindungen von Magneten mit einer Ampulle.

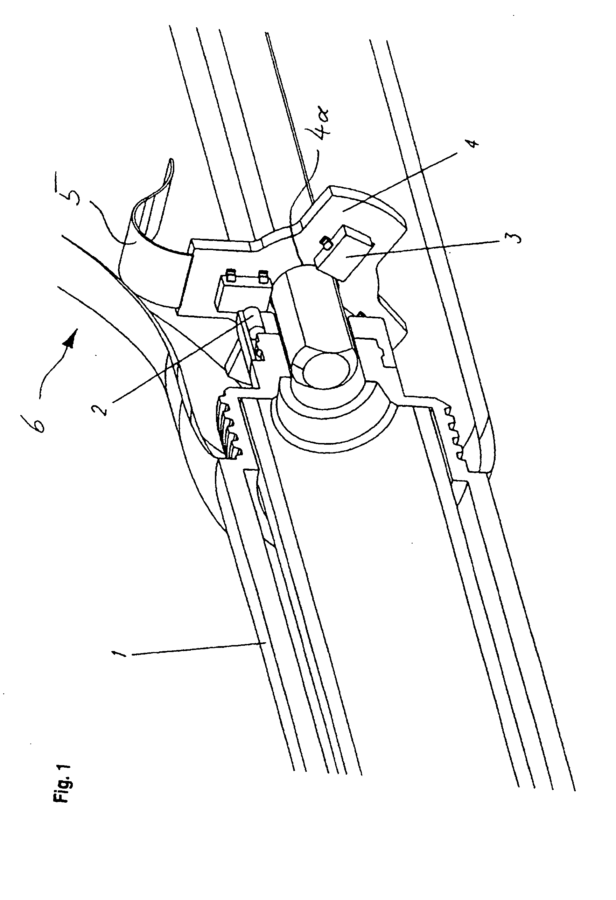

Figur 1 zeigt eine Ampulle 1 mit darin angeordneten stabförmigen Magneten 2, welche als Erkennungselemente dienen. Die Ampulle 1 ist vollständig in den Pen 6 eingeschraubt, so dass die zwei in der Ampulle 1 angeordneten Magnete 2 jeweils einem der vier asymmetrisch angeordneten Sensoren 3 gegenüberliegen, welche auf einer Platine 4 angeordnet und mit dem Pen 6 fest verbunden sind. Zum lagerichtigen Positionieren der Platine 4 mit den darauf angeordneten Sensoren 3 sind am Pen 6 Vorsprünge (nicht gezeigt) vorgesehen, welche in seitliche Ausnehmungen der Platine 4 eingreifen und somit die Platine 4 verdrehsicher im Pen 6 positionieren. Um die Signale der Sensoren 3 zu einer Auswerteelektronik übertragen zu können, ist eine flexible Leiterverbindung 5, welche mehrere nebeneinander liegende Leiter aufweist, mit der Platine 4 verbunden. Der Pen 6 weist weiterhin eine Batterie (nicht gezeigt) auf, mit welcher die Sensoren 3 und die Auswerteelektronik (nicht gezeigt) mit Strom versorgt werden können. Oberhalb eines Mechanikhalters, in dem zum Beispiel eine Gewindestange geführt wird, welche durch die Platine 4 bei einer zentralen Öffnung 4a hindurchtreten kann, ist eine Anzeigevorrichtung angeordnet, an welche zum Beispiel der mittels der Magneten 2 und Sensoren 3 detektierten Ampullen-Typ oder Ampulleninhalt dargestellt werden kann. Eine Feder zwischen Ampulle 1 und Platine 4 kann für einen stabilen Sitz der eingeschraubten Ampulle 1 sorgen.

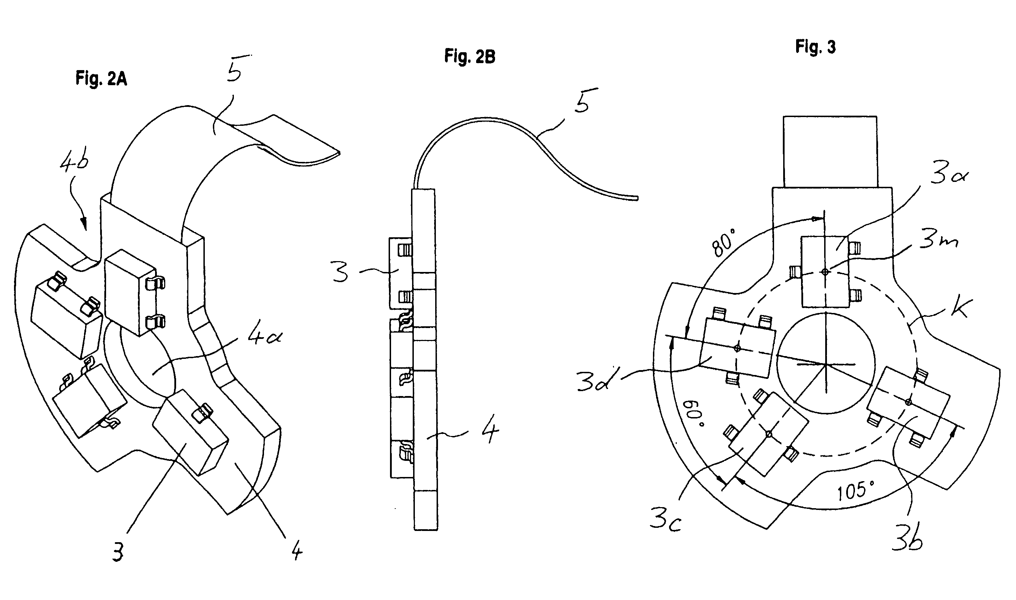

Figur 2A zeigt eine perspektivische Ansicht der in Figur 1 gezeigten Platine 4 mit darauf angeordneten Hall-Sensoren 3, wobei die Platine 4 seitliche Ausnehmungen 4b aufweist, welche in Verbindung mit den in Figur 1 gezeigten Vorsprüngen 10 zum korrekten Positionieren der Platine bzw. Leiterplatte 4 und damit der darauf angeordneten Hall-Sensoren 3 dient. Figur 2B zeigt eine Seitenansicht der in Figur 2A gezeigten Leiterplatte 4.

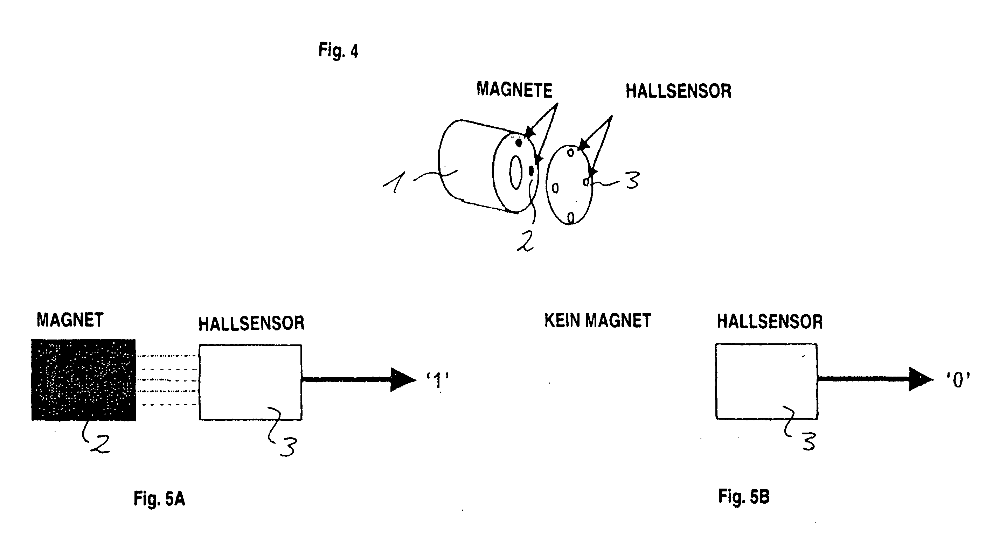

Figur 3 zeigt eine beispielhafte Ausführungsform der erfindungsgemäßen Anordnung von vier Hall-Sensoren 3 auf einer Leiterplatte 4. Dabei sind die Mittelpunkte 3m der Hall-Sensoren 3 entlang eines Kreises K angeordnet, welcher konzentrisch zur Mittelachse der daran anliegenden Ampulle 1 ist, wenn diese in den Pen 6 eingeschraubt ist. Zwischen dem Hall-Sensor 3a und dem Hall-Sensor 3b liegt ein Winkel von 115°, zwischen dem Hall-Sensor 3b und dem Hall-Sensor 3c liegt ein Winkel von 105°, zwischen dem Hall-Sensor 3c und dem Hall-Sensor 3d liegt ein Winkel von 60° und zwischen dem Hall-Sensor 3d und dem Hall-Sensor 3a liegt ein Winkel von 80°. Demzufolge sind die vier Hall-Sensoren 3a bis 3d asymmetrisch und nicht rotationssymmetrisch angeordnet und es kann eine fehlersichere Erkennung von Ampullen durchgeführt werden, wenn in den Ampullen den Hall-Sensor-Positionen entsprechend Positionen zur Aufnahme von als Erkennungselement dienenden Magneten 2 vorgesehen sind. Wird zum Beispiel die dem Hall-Sensor 3a entsprechende Position bei einer Ampulle immer mit einem Magneten 2 besetzt, so können drei verschiedene Ampullen-Typen eindeutig identifiziert werden, wenn ein erster Ampullen-Typ einen Magneten nur bei der dem Hall-Sensor 3b entsprechenden Position aufweist, ein zweiter Ampullen-Typ einen Magneten 2 bei der dem Hall-Sensor 3c entsprechenden Position aufweist und ein dritter Ampullen-Typ einen Magneten 2 bei der dem Hall-Sensor 3d entsprechenden Position aufweist.

Ist zum Beispiel ein Magnet beschädigt oder geht ein Magnet verloren, so gibt maximal einer der Hall-Sensoren 3a bis 3d ein Signal ab, so dass hieraus ermittelt werden kann, dass ein Fehler vorliegt. Wird eine Ampulle so eingeschraubt, dass ein Referenz-Magnet nicht wie erwünscht dem Hall-Sensor 3a gegenüberliegt, so kann es aufgrund der dargestellten asymmetrischen Anordnung der Sensoren 3a bis 3d zu keiner Konstellation kommen, bei welcher zwei an den vorgegebenen Positionen der Ampulle 1 angeordnete Magnete 2 jeweils einem Hall-Sensor 3 gegenüberliegen. Maximal wird nur einer der in der Ampulle 1 an den vorgegebenen Positionen angebrachten Magnete 2 einem der Sensoren 3a bis 3d gegenüberliegen.

Somit kann eine fehlersichere Detektion eines Ampullen-Typs bzw. des Inhalts einer bestimmten Ampulle erfolgen, wenn die Erkennungselemente 2 und Sensoren 3 erfindungsgemäß asymmetrisch angeordnet sind. Allgemein können die Signale von den vier Hall-Sensoren 3a bis 3d als ein digitales Code-Wort aufgefasst werden, wobei zur Erzeugung eines solchen Code-Wortes eine Sensoranordnung 3 einer Anordnung von Erkennungselementen 2 auf der Ampulle 1 gegenüberliegt, wie in Figur 4 gezeigt. Liegt einem einzelnen Sensor 3, wie zum Beispiel einem Hall-Sensor, ein Erkennungselement, also zum Beispiel ein Magnet 2 gegenüber, so kann dies beispielsweise als eine logische "1"aufgefasst werden, wie beispielhaft in Fig. 5A gezeigt, während bei Fehlen eines Magneten vor einem Hall-Sensor eine logische "0" ausgegeben wird, wie beispielhaft in Figur 5B gezeigt. Es können auch andere Erkennungselemente 2 statt der beispielhaft gezeigten Magnete mit entsprechenden Sensoren 3 verwendet werden.

Bei der beispielhaften Verwendung von vier Sensoren und zwei Erkennungselementen können sich demzufolge sechzehn mögliche Code-Wörter ergeben, wie in der nachfolgenden Tabelle gezeigt:

| **Code-Wort-Nr.** | **abcd** |
|---|---|
| 0 | 0000 |
| 1 | 0001 |
| 2 | 0010 |
| 3 | 0011 |
| 4 | 0100 |
| 5 | 0101 |
| 6 | 0110 |
| 7 | 0111 |
| 8 | 1000 |
| 9 | 1001 |
| 10 | 1010 |
| 11 | 1011 |
| 12 | 1100 |
| 13 | 1101 |
| 14 | 1110 |
| 15 | 1111 |

Dabei sind nur die Code-Worte 3, 5, 6, 9, 10 und 12 gültige Code-Worte, da alle anderen Code-Worte weniger oder mehr als zweimal eine logische "1" aufweisen. Wird - wie oben ausgeführt - zum Beispiel immer ein Erkennungselement so in der Ampulle 1 angeordnet, dass es einem bestimmten Sensor - im Beispiel der Figur 3 z. B. dem Sensor 3a - gegenüberliegt, so verringert sich die Anzahl der gültigen Code-Worte auf 3, nämlich die Code-Worte mit den Nummern 9, 10 und 12, bei welchen immer eine "1" an der Position a vorliegt. Ein solcher asymmetrischer Code liefert eine hohe Erkennungssicherheit bezüglich auftretender Fehler.

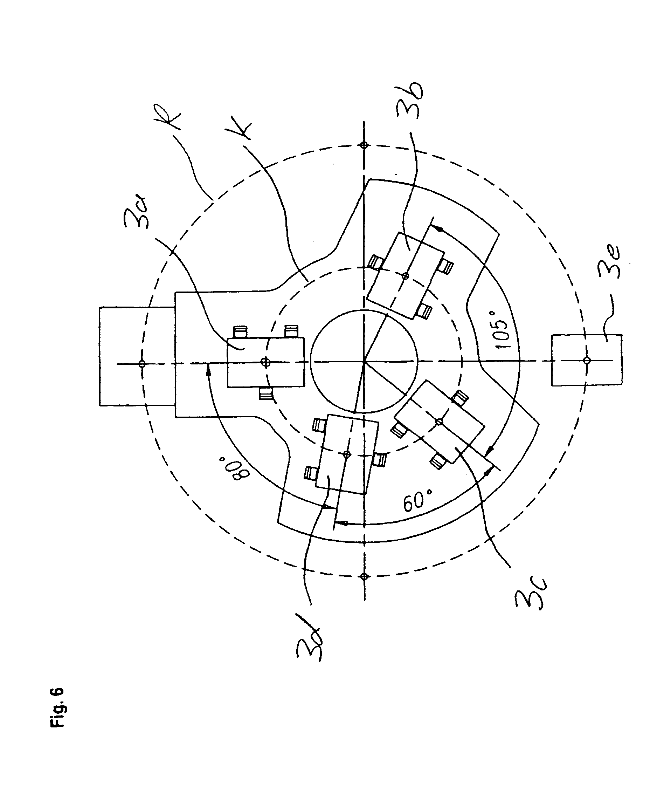

Es ist offensichtlich, dass die Erfindung auch bei anderen Codes als den hier beispielhaft nur zu Erläuterungszwecken angegebenen Codes verwendet werden kann. Figur 6 zeigte eine bevorzugte Ausführungsform einer erfindungsgemäßen Anordnung von Sensoren 3. Zusätzlich zu den Sensoren 3a bis 3d, deren Anordnung der in Figur 3 beschriebenen Anordnung entspricht, ist in dem gezeigten Ausführungsbeispiel ein weiterer Hall-Sensor 3e vorgesehen, welcher außerhalb des Kreises K liegt, auf dem die Mittelpunkte der Sensoren 3a bis 3d liegen. Werden zum Beispiel in einer in eine Verabreichungsvorrichtung einzuschraubenden Ampulle 1 mehrere Erkennungselemente so angeordnet, dass sie sich in etwa auf einem Kreis R befinden, auf welchem der Mittelpunkt des weiteren Hall-Sensors 3e angeordnet ist, so kann zum Beispiel beim Einschrauben der Ampulle von Hand in den Pen 6 mittels beispielsweise vier Magneten 2, welche auf dem Kreis R im Winkelabstand von 90° angeordnet sind, eine Drehbewegung der Ampulle 1 erkannt werden und in Verbindung mit der oben beschriebenen Anordnung von Magneten 2 in der Ampulle 1 zusammen mit der beispielhaft gezeigten Anordnung der Hall-Sensoren 3a bis 3d kann bereits beim Einschrauben eine Lageerkennung der Magnete 2 erfolgen, welche in den Hall-Sensoren 3a bis 3d korrespondierenden Positionen angebracht sind.

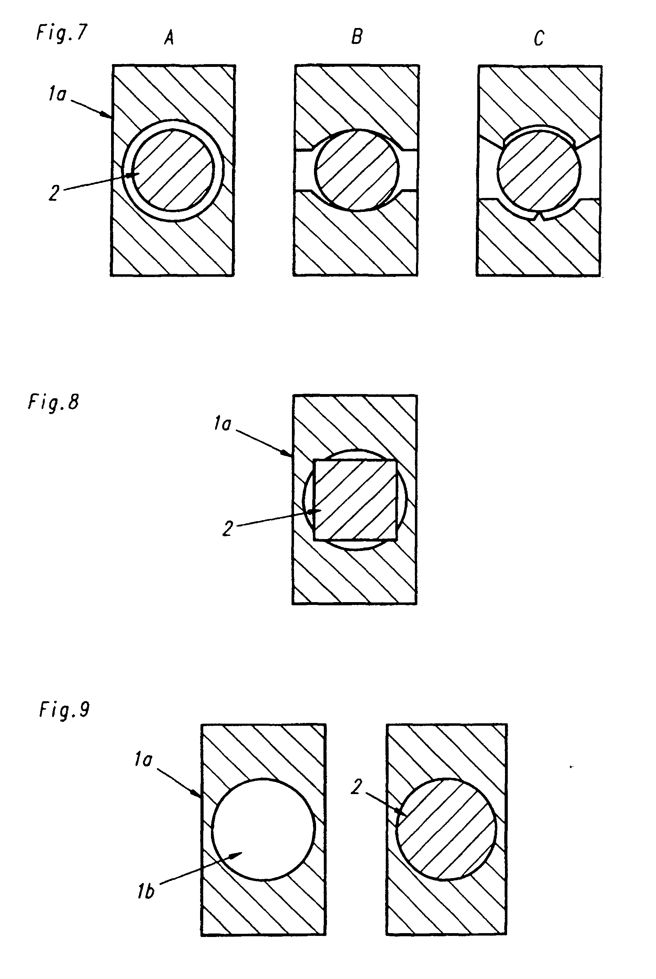

Die Figuren 7 bis 9 zeigen beispielhaft mögliche Anordnungen von Magneten oder anderen Erkennungselementen in der Ampulle 1, insbesondere im vorderen Bereich des Ampullenumfangs, jedoch können auch andere der oben beschriebenen Erkennungselemente anstatt der Magnete 2 entsprechend angebracht werden.

Figur 7 zeigt drei Ausführungsbeispiele zum Anbringen eines Erkennungselements 2, z. B. eines Magneten in der Wand 1 a einer Ampulle 1, wobei der Magnet 2 zylinderförmig ausgebildet ist und als kreisrunder Querschnitt gezeigt ist. Dabei können bei der in Figur 7A gezeigten Ausführungsform in einem zylinderförmigen Hohlraum Magnete 2 eingebracht werden, welche eine gewisse Toleranz in ihren Abmessungen aufweisen, wobei die Magnete 2 in den entsprechenden Hohlräumen entweder frei gelagert oder durch ein geeignetes Klebemittel fixiert werden können. Die Figuren 7B und 7C zeigen die Befestigung eines Magneten 2 durch Verklemmen in der Ampullenwand 1 a. Dabei ist es vorteilhaft, wenn die Ampullenwand 1 a aus einem elastischen Material, wie zum Beispiel einem geeigneten Kunststoff besteht.

Figur 8 zeigt eine alternative Ausführungsform zur Befestigung eines Magneten 2 in einer Ampullenwand 1a, wobei der Magnet 2 quaderförmig ausgebildet ist und deformiert werden kann. Wird ein solcher quaderförmiger Magnet 2 an seinen Kanten deformiert und in einen zylinderförmigen Hohlraum der Ampullenwand 1a eingebracht, so kann sich der Magnet 2 durch seine Kanten seitlich in der Ampullenwand 1 a abstützen und somit fest in der Ampullenwand 1 a halten.

Figur 9 zeigt eine weitere Ausführungsform zum Ausbilden eines Magneten 2 in einer Ampullenwand 1a, wobei eine Kunststoffemulsion mit magnetischen oder magnetisierbaren Materialien in einen zylinderförmigen Hohlraum 1b der Ampullenwand 1 a eingebracht wird.

## Patentansprüche

1. Ampulle für ein Injektions- oder Infusionsgerät mit einer Erkennungseinrichtung, die mindestens zwei Erkennungselemente (2) umfasst, die jeweils in einer von mindestens drei vorgegebenen Positionen an der Ampulle (1) angeordnet werden können, **dadurch gekennzeichnet, dass** die mindestens drei vorgegebenen Positionen auf der Ampulle (1) derart um eine Mittelachse der Ampulle (1) angeordnet sind, dass ein Winkelabstand zwischen jeweils zwei Positionen ungleich einem Winkelabstand zwischen zwei anderen Positionen ist.

2. Ampulle nach Anspruch 1, wobei die mindestens drei vorgegebenen Positionen oder die mindestens zwei Erkennungselemente (2) an einem Ende der Ampulle (1), bevorzugt auf einem Kreis (K) angeordnet sind, welcher konzentrisch zur Mittelachse der Ampulle (1) ist.

3. Ampulle nach einem der vorhergehenden Ansprüche, wobei drei, vier, fünf bis fünfzig vorgegebene oder mehr als fünfzig vorgegebene Positionen vorgesehen sind, bei welchen die mindestens zwei Erkennungselemente (2) angeordnet werden können und drei bis fünfzig oder mehr als fünfzig Erkennungselemente (2) vorgesehen sind.

4. Ampulle nach einem der vorhergehenden Ansprüche, wobei mindestens eines der mindestens zwei Erkennungselemente (2) an einer bestimmten vorgegebenen Position als ReferenzElektrode angeordnet ist.

5. Ampulle nach einem der vorhergehenden Ansprüche, wobei die mindestens zwei Erkennungselemente (2) auf einem elektrischen und/oder magnetischen bzw. induktiven und/oder kapazitiven und/oder mechanischen Prinzip basieren, bevorzugt Magnete, leitfähige Strukturen, optische Strukturen oder Oberflächenstrukturen sind.

6. Ampulle nach einem der vorhergehenden Ansprüche, wobei die mindestens zwei Erkennungselemente (2) unterschiedlich starke elektrische und/oder magnetische Felder erzeugen.

7. Verabreichungsvorrichtung, welche mit einer Ampulle nach einem der Ansprüche 1 bis 10 koppelbar ist und mindestens zwei Sensoren (3) aufweist, welche an vorgegebenen Positionen angeordnet werden können, um die Anordnung der mindestens zwei Erkennungselemente (2) an den vorgegebenen Positionen der Erkennungselemente zu erkennen, wobei die vorgegebenen Positionen der Sensoren den vorgegebenen Positionen der Erkennungselemente gegenüberliegen.

8. Verabreichungsvorrichtung nach Anspruch 7 mit einem auf die Ampulle (1) ausgerichteten Gewinde und/oder einem Endanschlag zum Definieren einer Endposition der eingeschraubten Ampulle (1).

9. Verabreichungsvorrichtung nach Anspruch 7 oder 8 mit einer Vorrichtung zum Übermitteln von Signalen zwischen den Erkennungselementen (2) und den Sensoren (3).

10. Verabreichungsvorrichtung nach einem der Ansprüche 7 bis 9, wobei die Sensoren Hall-Sensoren, optische Sensoren, elektrische Sensoren und/oder mechanische Sensoren, insbesondere Kontaktschafter sind.

11. Verabreichungsvorrichtung nach einem der Ansprüche 7 bis 10 mit einer Anzeigevorrichtung (9) zum Anzeigen eines von den Sensoren (3) detektierten Ampullen-Typs.

12. System mit einer Ampulle nach einem der Ansprüche 1 bis 6 und einer Verabreichungsvorrichtung nach einem der Ansprüche 7 bis 11.

## Claims

1. An ampoule for an injection or infusion device comprising a recognition device which includes at least two recognition elements (2) which can be respectively arranged in one of at least three predetermined positions on the ampoule (1), **characterised in that** the at least three predetermined positions on the ampoule (1) are arranged around the central axis of the ampoule (1) in such a way that an angular spacing between two respective positions is unequal to an angular spacing between two other positions.

2. An ampoule according to claim 1 wherein the at least three predetermined positions or the at least two recognition elements (2) are arranged at one end of the ampoule (1), preferably on a circle (K) which is concentric with respect to the central axis of the ampoule (1).

3. An ampoule according to one of the preceding claims wherein there are provided three, four, five to fifty predetermined positions or more than fifty predetermined positions at which the at least two recognition elements (2) can be arranged and there are provided three to fifty or more than fifty recognition elements (2).

4. An ampoule according to one of the preceding claims wherein at least one of the at least two recognition elements (2) is arranged at a given predetermined position as a reference electrode.

5. An ampoule according to one of the preceding claims wherein the at least two recognition elements (2) are based on an electrical and/or magnetic or inductive and/or capacitive and/or mechanical principle, and are preferably magnets, conductive structures, optical structures or surface structures.

6. An ampoule according to one of the preceding claims wherein the at least two recognition elements (2) generate electrical and/or magnetic fields of different strengths.

7. An administration device which can be coupled to an ampoule according to one of claims 1 to 6 and has at least two sensors (3) which can be arranged at predetermined positions in order to recognise the arrangement of the at least two recognition elements (2) at the predetermined positions of the recognition elements, wherein the predetermined positions of the sensors are in opposite relationship to the predetermined positions of the recognition elements.

8. An administration device according to claim 7 with a screwthread aligned with the ampoule (1) and/or an end abutment for defining an end position of the screwed-in ampoule (1).

9. An administration device according to claim 7 or claim 8 with a device for communicating signals between the recognition elements (2) and the sensors (3).

10. An administration device according to one of claims 7 to 9 wherein the sensors are Hall sensors, optical sensors, electrical sensors and/or mechanical sensors, in particular contact switches.

11. An administration device according to one of claims 7 to 10 with a display device (9) for displaying an ampoule type detected by the sensors (3).

12. A system comprising an ampoule according to one of claims 1 to 6 and an administration device according to one of claims 7 to 11.

## Revendications

1. Ampoule pour un appareil d'injection ou de perfusion pourvu d'un dispositif d'identification, qui comprend au moins deux éléments d'identification (2), qui peuvent être disposés respectivement dans une des au moins trois positions prédéfinies sur l'ampoule (1), **caractérisée en ce que** les au mois trois positions prédéfinies sur l'ampoule (1) sont disposées autour de l'axe central de l'ampoule (1) de manière à ce qu'une distance angulaire entre respectivement deux positions soit différente de la distance angulaire entre deux autres positions.

2. Ampoule selon la revendication 1, dans laquelle les au moins trois positions prédéfinies ou les au moins deux éléments d'identification (2) sont disposés à une extrémité de l'ampoule (1), de préférence sur un cercle (K), lequel est disposé de manière concentrique par rapport à l'axe de l'ampoule (1).

3. Ampoule selon l'une quelconque des revendications précédentes, dans laquelle trois, quatre, cinq jusqu'à cinquante positions prédéfinies ou plus sont prévues, dans lesquelles les au moins deux éléments d'identification (2) peuvent être disposés et dans laquelle de trois à cinquante éléments d'identification (2) ou plus sont prévus.

4. Ampoule selon l'une quelconque des revendications précédentes, dans laquelle au moins un des au moins deux éléments d'identification (2) est disposé à une position prédéfinie indiquée comme électrode de référence.

5. Ampoule selon l'une quelconque des revendications précédentes, dans laquelle les au moins deux éléments d'identification (2) se basent sur un principe électrique et/ou magnétique ou inductif et/ou capacitif et/ou mécanique, qui sont de préférence des aimants, des structures conductrices, des structures optiques ou des structures de surface.

6. Ampoule selon l'une quelconque des revendications précédentes, dans laquelle les au moins deux éléments d'identification (2) génèrent des champs électriques et/ou magnétiques de puissance différente.

7. Dispositif d'administration, lequel peut être couplé avec une ampoule selon l'une quelconque des revendications 1 à 10 et comporte au moins deux capteurs (3), qui peuvent être disposés à des positions prédéfinies, pour identifier la disposition des au moins deux éléments d'identification (2) aux positions prédéfinies des éléments d'identification, les positions prédéfinies des capteurs étant superposées aux positions prédéfinies des éléments d'identification.

8. Dispositif d'administration selon la revendication 7 pourvu d'un filetage aligné sur l'ampoule (1) et/ou d'une butée finale pour définir une position finale de l'ampoule (1) vissée.

9. Dispositif d'administration selon la revendication 7 ou 8 pourvu d'un dispositif de transmission de signaux entre les éléments d'identification (2) et les capteurs (3).

10. Dispositif d'administration selon l'une quelconque des revendications 7 à 9, dans lequel les capteurs sont des capteurs de Hall, des capteurs optiques, des capteurs électriques et/ou des capteurs mécaniques, en particulier des contacteurs.

11. Dispositif d'administration selon l'une quelconque des revendications 7 à 10 pourvu d'un dispositif d'affichage (9) destiné à afficher un type d'ampoule détecté par les capteurs (3).

12. Système pourvu d'une ampoule selon l'une quelconque des revendications 1 à 6 et d'un dispositif d'administration selon l'une quelconque des revendications 7 à 11.
